# FASCICULE DE BREVET EUROPEEN

(11) **EP 0 537 089 B1**
(45) Date de publication et mention de la délivrance du brevet: **13.05.1998**
(21) Numéro de dépôt: 92420348.2
(22) Date de dépôt: 02.10.1992
(51) Int. Cl.: C07K 1/06

(54) **Réactif utile pour le clivage et procédé d'utilisation de ce réactif**
Reagenzzusammensetzung zur Deproduktion und ihre Verwendung
Reagent composition for deprotection, and its use

(30) Priorité: 11.10.1991 FR 9112524
(43) Date de publication de la demande: 14.04.1993
(73) Titulaire: RHODIA CHIMIE, 92408 Courbevoie Cédex (FR)
(72) Inventeur: Pevere, Virginie, F-69008 Lyon (FR)
(74) Mandataire: Ricalens, François

(56) Documents cités:
- INTERNATIONAL JOURNAL OF PEPTIDE AND PROTEIN RESEARCH vol. 36, 1990, COPENHAGUE, DANEMARK pages 255 - 266 KING ET AL 'A cleavage method which minimizes side reactions following Fmoc solid phase peptide synthesis'
- EDITEUR & AUTEUR: EPTON,J 'Solid Phase Synthesis // First Intl. Symp; Aug 29 Sept 2, 1989; Oxford & Deprotection studies in ultra-high load solid (gel) phase peptide synthesis' 1990 , SPCC (UK) LTD , BIRMINGHAM, ANGLETERRE
- TETRAHEDRON LETTERS vol. 29, no. 3, 1988, OXFORD pages 303 - 306 KAISER ET AL 'Chlorotrimethylsilane-phenol as a mild deprotection reagent for the tert-butyl based protecting groups in peptide synthesis'
- CHEMICAL ABSTRACTS, vol. 101, 1984, Columbus, Ohio, US; abstract no. 171728, BODANSZKY 'Improved selectivity in the removal of the tert-butyloxycarbonyl group' page 746 ;colonne 1 ;

## Description

La présente invention a pour objet un réactif et un procédé pour cliver les fonctions protégées par un groupe alcoxy-carbonyle lors de la synthèse organique. Il est courant de protéger une molécule en bloquant les fonctions qui dans les conditions opératoires visées seraient réactives ou réputées telles par des groupes qualifiés de protecteur.

Ces techniques sont particulièrement utiles lors des synthèses peptidiques et les fonctions les plus couramment protégées sont les fonctions amines, alcool ou thiol.
Parmi les groupes les plus couramment utilisés, on peut citer le groupe BOC ou terbutyloxycarbonyle, les groupes Z ou benzyloxyle, voire même le groupe FMOC.

La déprotection usuellement utilisée est une lyse en milieu acide en général en milieu halohydrique anhydre (c'est-à-dire avec une teneur en eau en général inférieure à 1%, avantageusement à 10⁻³, de préférence à 10⁻⁴).

Toutefois cette technique présente de nombreux inconvénients. La réaction de clivage est parfois lente ou nécessite de gros excès de réactif. Les groupements alcoxyle ont tendance à se transformer en carbocation avec évolution vers les double liaisons lorsque cela est possible, ou vers des réactions d'alcoylation sur le noyau, ce qui est particulièrement gênant dans le cas des synthèses de peptides dont la séquence présente des résidus nucléophiles tels que des noyaux aromatiques (tryptophane, tyrosine, phénylalanine,....) ou soufrés (méthionine).

Récemment King et al. (Int. J. Peptide Protein Res, 1990, page 255-266) ont proposé d'utiliser un mélange aqueux d'acide trifluoroacétique de phénol et de dérivés soufrés (thioanisole et EDT [éthane dithiol 1,2]). Toutefois ce mélange reste complexe et l'usage des composés soufrés né pas toujours aisé.

C'est pourquoi, un des buts de la présente invention est de fournir un procédé et un réactif qui accélèrent sensiblement la cinétique.
Un autre but de la présente invention est de fournir un réactif et un procédé qui permettent de privilégier la formation d'halogénure ou de pseudo-halogénure, vis-à-vis de la formation d'insaturé(s).
Un autre but de la présente invention est de fournir un procédé et un réactif qui évitent les réactions d'alcoylation des noyaux aromatiques.

Plus généralement un des buts de la présente invention est de fournir un procédé permettant de piéger des espèces de type carbocation, avec une éventuelle évolution vers la formation d'halogènure ou de pseudo- halogènure. Ces buts et d'autres sont atteints au moyen d'un réactif utile notamment pour cliver les groupes alcoxy-carbonyle des fonctions qu'il protège caractérisé par le fait qu'il comporte :
- un phénol B ;
- un diluant A ;
- un acide halohydrique ou pseudo-halohydrique dont l'acidité exprimée par la constante de Hammett est au moins égale à celle de l'acide formique, de préférence au moins égale à celle de l'acide chlorhydrique.
La teneur en eau est aussi faible que possible avantageusement, en masse, au plus égale à3 % de préférence aux valeurs mentionnée plus haut, dans l'exposé du problème.
La teneur en acide est en général, et notamment pour les préférés, limitée par la solubilité de l'acide dans le milieu ; lorsque cela n'est pas le cas on ne dépasse pas 10% en masse de la somme diluant A plus phénol B.
La quantité de phénol B est de préférence d'au moins 1/200 du diluant A.
Il n'existe pas de limite supérieure stricte. Ainsi, l'utilisation de phénol B (produit pur ou mélange) sans diluant A, fait partie de l'invention dans la mesure où les conditions suivantes sont remplies :
- Le point de fusion du phénol B est au plus égal à 50 °C (dans la présente description les zéros ne sont pas des chiffres significatifs sauf si cela est spécifié autrement) ;
- Le phénol B peut être séparé d'avec le produit réactionnel par distillation éventuellement ou pression réduite ;
- Le phénol B n'est pas miscible à l'eau en toute proportion ; avantageusement il ne dissout au plus que 10% en poids de préférence 5%, plus préférentiellement 1 % d'eau ;
- Il est également préférable que l'eau ne puisse dissoudre qu'au plus 10 % de phénol B, avantageusement au plus 1 % en masse et ce même en présence du diluant A en tant que tiers solvant.
- Le phénol B ne forme pas d'émulsion stable susceptible de gêner l'élimination des co-produits par lavage à l'eau.
Les halogéno phénols avantageusement di-, et de préférence monochloro, ainsi que les alcoylphénols légers répondent par exemple à ces contraintes.
Il est toutefois préférable qu'il y ait un diluant A et un phénol B.

Avantageusement, le rapport massique entre ledit phénol B et ledit diluant A est compris entre 1/200 et 1, de préférence entre 1/20 et 1/2.
Les valeurs ci-dessus exprimées en rapport massique conviennent bien pour le phénol lui-même et pour les phénols dont la masse moléculaire n'est pas d'un ordre de grandeur différent de celui du phénol proprement dit(C₆H₅OH).

Pour les phénols de poids moléculaires élevés, la concentration en mole par litre est au moins égale à 5.10⁻², avantageusement entre 0,1 et 2 de préférence entre 0,5 et 1,5 M.
Lorsque les diluants A et les phénols B ne sont pas miscibles en toute proportion, la limite supérieure de la teneur en phénol(s) est la plus basse des deux limites : celle mentionnée ci-dessus et la limite de solubilité.
Par l'expression phénol B, il faut comprendre des phénols ou des mélanges de phénols.
Les diluants A sont des solvants organiques suffisamment polaires pour dissoudre au moins 1 % de préférence au moins 2 % en masse du phénol "stricto sensu" (C₆ H₅ OH) et sont suffisamment hydrophobes pour n'être pas miscible à l'eau en toute proportion.
Il est préférable que l'eau ne puisse dissoudre qu'au plus 10 % de preférence au plus 5% de diluant A, avantageusement au plus 1 % en masse et ce même en présence du phénol B en tant que tiers solvant.
Il sont de préférence peu basiques c'est-à-dire que leur indice donneur (ou de nucléophilicité tel que défini dans l'ouvrage de REICHARDT C. Solvent Effect in Organic Chemistry Verlag Chemie, Weinheim, New York 1979; b.
Reichardt, C. and Harbusch-Görnert, E. Justus Liebigs Ann. Chem. (1983) 721. soit au plus égal de préférence inférieur à environ celui du tétrahydrofuranne. L'indice donneur est en principe égal à moins la valeur de l'enthalpie exprimée en Kcal/moles de l'interaction entre le solvant et l'antimoine en solution de chlorure de méthylène dilué (cf. 6 Gutmann, V. Electrochim. Acta 21 (1976) 661 ; Gutmann, V. and Wychera, E. Inorg. Nucl. Chem. Lett. 2 (1966) 297 ; Mayer, V. and Gutmann, V. Monatsh. Chem. 101 (1970) 912.
Bien entendu les diluants ne doivent pas être des acides protiques ainsi leur PKa ne doit avantageusement pas être iférieur à 15 de préférence à 20 plu préférentiellement à 30.
Les diluants A peuvent être des mélanges, y compris des fractions pétrolières. Naturellement, dans les conditions opératoires les diluants A doivent être inertes vis-à-vis des phénols et des réactifs de synthèse peptidique utilisés.

Les familles préférées de diluants sont choisis dans le groupe constitué par les dérivés aromatiques, les éthers, les esters et les solvants halogénés.

A titre de paradigmes de membres de ces familles on peut citer comme dérivés aliphatiques halogénés le dichlorométhane, le dichloro- 1,2-éthane, le trichloro-1,1,1-éthane, comme dérivés aromatiques le toluène et comme dérivés aromatiques halogénés le chlorobenzène, comme esters l'acétate d'éthyle et l'acétate d'isopropyle, comme éthers, l'oxyde de tertiobutyle et de méthyle ainsi que l'anisole.
Pour des raisons d'économie industrielle, il est préférable que le diluant A soit distillable sous pression atmosphérique ou sous vide primaire ou secondaire. En général le phénol B est choisi dans le groupe des composés répondant à la formule suivante I.

(R₁)n - Aᵣ - O - H (I)

dans laquelle :
- Aᵣ représente un radical aromatique monocyclique, polycyclique, hétérocyclique ou non ;
- Les substituants R₁, semblables ou différents, représentent :
   . un motif halogène, de préférence fluor, chlore, Brome
   . un groupe -Z-R₂, où Z peut être
      - une simple liaison;
      - un atome d'oxygène;
   où R₂ représente un atome d'hydrogène, un radical alcoyle ou aryle éventuellement hydroxylés ou mono ou poly halogénés d'au plus 8 atomes de carbone,
- n représente le nombre de substituants et est égal à 0 ou à un entier au plus égal au nombre de positions substituables sur les noyaux aromatiques ; et de leurs mélanges.

Les groupes alcoyles (tels que définis étymoliquement ou dans le dictionnaire de la Chimie Duval Presses Scientifiques Internationale PARIS Vle 1959) peuvent notamment être des restes aliphatiques linéaires ou ramifiés d'au plus 6 atomes de carbone ou des restes arylaliphatiques.

Le nombre de positions substituables est déterminable aisément à l'aide de règles simples connues de l'homme de métier.

Ainsi par exemple :
- quand: Aᵣ = phényle n < 5
Aᵣ = naphtyle < 7

Avantageusement le phénol B a un nombre d'atomes de carbone d'au plus 30 atomes de carbone, de préférence au plus 20 atomes de carbone.
Avantageusement le phénol n'est pas basique, c'est-à-dire que lorsque il y a hétéroatome dans le cycle, ledit hétéroatome n'est pas choisi dans la colonne VB de la classification périodique des éléments (cf. Bull. Soc. Chim. supplément au numéro 1 de janvier 1966).
Il est souhaitable que les positions vicinales de la fonction phénol soient non substituées ou occupées par des groupes non encombrants. Sont considérés comme encombrants les radicaux reliés aux dites positions vicinales par un carbone tertiaire voire secondaire.
Les composés mono cyclique sont ceux qui donnent le meilleur compromis efficacité-coût; on préfère ceux à 6 chaînons (noyau pyridinyle ou phényle).
Il est également souhaitable que Z-R₂ ne puissent signifier plus de trois fois de préférence plus de 2 fois un groupe hydroxyle.

Avantageusement, dans la formule I, les radicaux R sont choisis parmi le groupe constitué par :
- les radicaux méthyle, éthyle, propyles, butyles,
- les radicaux trifluorométhyle et pentafluoroéthyle,
- les radicaux méthoxyle, éthoxyle, propyloxyles, butyloxyles,
- phényle, hydroxyphényles, et Ar-OH,
- les radicaux phényloxyles, hydroxyphényloxyles,
- atomes de fluor, de chlore et de brome.

Pour ne pas trop alourdir la molécule de phénol, il est souhaitable que dans la formule I n soit au plus égal à 5.
Parmi les phénols donnant les meilleurs résultats, il convient de citer :
- les mono halogéno phénols (de préférence mono chloro),
- les poly halogéno phénols, (de préférence polyfluoro),
- les phénols mono ou disubstitués par des radicaux alkyles de C₁ à C₄, alkoxy de C₁ à C₄, des perfluoroalkyle de C₁ à C₂ et le 2, 2, 2-trifluoro éthyle,
- les diphénols,
- le phénol au sens étroit,
- les naphtols éventuellement mono ou di- substitués.
Il est préférable que la quantité de phénol soit au moins égal à celle qui correspond à une molécule de phénol par motif d'acide aminé; avantageusement à trois.
L'acide aminé peut être C ou N protégé; dans ce dernier cas, le groupement acide peut être activé sur la fonction acide, par les fonctions acide latérale protégé, les acides aminés peuvent être naturels ou synthétiques.
Le milieu selon la présente invention permet également de dissoudre un peptide protégé.

L'acide halohydrique préféré est choisi parmi l'acide bromhydrique, iodhydrique, de préférence chlorhydrique et leurs mélanges.
On peut envisager également les acides correspondants aux pseudo-halogènes tel que l'acide trifluorométhanesulfonique (triflique). Les groupes alcoxy-carbonyle peuvent être écrits sous la forme R-O-CO- où R représente avantageusement un groupe alcoyle y compris aralcoyle, alcoyle ayant la définition du dictionnaire de Chimie DUVAL Presse Scientifique internationale, Paris Vle, 1959.
Le groupe alcoyle représente de préférence un groupe alkyle ayant au plus 15 atomes de carbone de préférence au plus 7 atomes de carbone. Le groupe R est un groupe de préférence secondaire, avantageusement tertiaire, tel que le groupe tertiobutyle qui est un des radicaux préférés. Le radical peut également être un groupe aralkyle qui comporte au plus 10 noyaux de préférence de 1 à 8 noyaux.
On peut citer notamment le groupe benzyle, le groupe FMOC ou les groupes décrits dans la demande de Brevet Britannique déposée le 31/12/1990 déposée sous le n° 90 28208.8. La concentration en acide halohydrique est avantageusement maintenue à une valeur au moins égale à la solubilité du gaz dans le milieu en équilibre avec une pression partielle moins égale à 10³, de préférence à 10⁴ Pascals, en général à celle obtenue par barbotage dudit acide halohydrique sous pression atmosphérique. On peut également opérer sous pression plus élevée.
Dans la mesure où l'on peut indiquer une fourchette, on peut considérer que exprimée en mole(s) par litre, il est souhaitable que la concentration en acide et donc la solubilité soit au moins égale à 10⁻⁴ M de préférence comprise entre 10⁻³ et 10⁻¹ M.
La température n'a pas besoin d'être très élevée et on peut donner à titre indicatif une température comprise entre la température de fusion commençante du milieu et 100°C environ, ou lorsque cette température est inférieure à 100°C, la température d'ébullition du système réactionnel.
Cette technique est particulièrement bien adaptée aux synthèses des peptides notamment des oligopeptides de 2 ou 3 à 50, de préférence de 5 à 25 acides aminés.

D'une manière générale le système de solvants permet au(x) carbocation(s) engendré(s), notamment R^{+,} par l'action sur un substrat ou un réactif de l'acide ou halo- ou pseudohalohydrique d'évoluer vers une forme halogénure ou un pseudohalogénure. Ainsi le réactif est-il également utile pour synthétiser les (pseudo)halogénures à partir de composés permettant la génération de carbocation (correspondant au halogénures désirés) dans un milieu acide anhydre.
sans que la déposante soit liée par une telle explication les inventeurs pensent au passage par un intermédiaire éther de phénol (peu stable dans les conditions opératoires mais présent à des niveaux significatifs au cours de la réaction).
Ainsi le présent réactif peut être utile pour synthétiser des éthers mixtes de phénols avec le radical R
Parmi les phénols utilisables un mention doit être faite des phénols pentasubstitués tel que les pentahalogènophénols (avec pour les halogènes les mêmes préférences que celle évoquée plus haut) de manière à éviter toute dégradation du phénol par action sur ce dernier du carbocation.
Les exemples non limitatifs suivants illustrent l'invention.

### EXEMPLES

### MODE OPERATOIRE

Un oligo-peptide protégé est dissous dans un mélange chlorure de méthylène et un hydroxy aromatique. On soumet ce mélange réactionnel au barbotage de l'acide chlorhydrique gazeux jusqu'au moment où l'on est plus capable de déceler l'existence de l'oligo-peptide protégé.

### Exemple 1

La réaction est menée sur l'oligo-peptide BOC PheLeu OGPC dans le chlorure de méthylène en présence de phénol, en présence d'orthocrésol ou sans rien du tout. Les résultats sont rassemblés dans le tableau suivant :
Résultats du clivage
Modèle BocPheLeu OGPC
GPC : groupement O-protecteur de type benzylique.
Déprotection à température ambiante barbotage HCl (débit 1l/h).

| % de Ar-OH dans CH₂ Cl₂ | temps de clivage |
|---|---|
| zéro | 60 mn |
| **F-OH** (10 %) | 30 mn |
| **CH**_{**3**}**-F-OH** (10 %) | 30 mn |

### exemple 2 Déprotection en présence de phénol (ou ortho-crésol)

Il a été démontré que HCl est très peu soluble dans un mélange CH₂Cl₂ - ArOH (10 %) ;
Avec phénol (10 % dans CH₂Cl₂) :
- après 40 mn de barbotage HCl, dosage d'acidité :
   1er dosage : 0,06 M/l HCl dans le solvant ;
   2ème dosage : 0,03 M/l HCl dans le solvant.
L'accélération de la vitesse de réaction ne s'explique pas par une plus grande solubilisation HCl dans CH₂Cl₂.

Différence de mécanisme.
Au cours du clivage, la présence de l'intermédiaire Ar-O-C(CH₃)₃ a été décelée.

Celui-ci ne s'accumule pas dans le milieu réactionnel : il est lui-même clivé par HCl.

Cette réaction semble rapide :

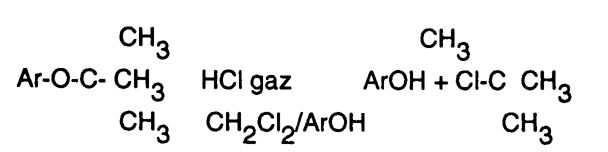

D'autres phénols tels que le pentafluorophénol donnent également de bons résultats

## Revendications

1. Réactif anhydre utile notamment pour cliver les groupes alcoxy-carbonyle des fonctions qu'il protège comportant :
- un diluant A ;
- un acide halohydrique ou pseudo-halohydrique dont l'acidité est au moins égale à celle de l'acide formique,
caractérisé par le fait qu'il comporte en outre :
- un phénol B dans un rapport massique avec ledit diluant d'au moins 1/200 ; avantageusement compris entre 1/200e et 1, de préférence entre 1/20e et 1/2 ;
et par le fait que ledit diluant présente les caractéristiques suivantes :
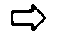 capable de dissoudre au moins 1 %, avantageusement 2 % du phénol stricto sensu ;
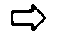 non miscible en toute proportion avec l'eau ;
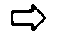 Inerte.

2. Réactif selon la revendication 1, caractérisé par le fait que ledit diluant A est tel que l'eau ne puisse dissoudre qu'au plus 10 % de preférence au plus 5% dudit diluant A.

3. Réactif selon les revendications 1 et 2, caractérisé par le fait que ledit phénol est choisi dans le groupe des composés répondant à la formule générale suivante I :
(R₁)n-Ar-O-H (I)
dans laquelle :
- Ar représente un radical aromatique monocyclique, polycyclique, hétérocyclique ou non ;
- Les substituants R₁, semblables ou différents, représentent :
- un motif halogène, de préférence fluor, chlore, brôme :
- un groupe -Z-R₂-, où Z peut être
- une simple liaison ;
- un atome d'oxygène ;
où R₂ représente un atome d'hydrogène, un radical alcoyle ou aryle éventuellement hydroxylés ou mono ou poly halogénés d'au plus 8 atomes de carbone,
- n représente le nombre de substituants et est égal à 0 ou à un entier au plus égal au nombre de positions substituables sur les noyaux aromatiques et de leurs mélanges.

4. Réactif selon l'une des revendications 1 à 3, caractérisé par le fait que ledit phénol B a un nombre d'atomes de carbone d'au plus 30 atomes de carbone de préférence au plus 20 atomes de carbone.

5. Réactif selon l'une des revendications 1 à 4, caractérisé par le fait que les positions vicinales de la fonction phénol sont non substituées ou occupées par des groupes non encombrants.

6. Réactif selon l'une des revendications 1 à 5 caractérisé par le fait que le radical - Ar- est un aromatique monocyclique ayant de préférence 6 chaînons.

7. Réactif selon les revendications 1 à 6 caractérisé par le fait que ledit diluant A est choisi parmi les aromatiques les éthers, les esters et les solvants halogénés.

8. Réactif selon l'une des revendications 1 à 7 caractérisé par le fait que l'acide halohydrique est choisi parmi les acides chlorhydrique, bromhydrique, iodhydrique, de préférence chlorhydrique.

9. Procédé de traitement des molécules protégées caractérisé par le fait que ladite molécule est protégée par un groupement alcoxy-carbonyle et par le fait que ladite molécule est soumise aux réactifs selon l'une des revendications 1 à 8.

10. Procédé selon la revendication 9, caractérisé par le fait que ladite molécule protégée est un oligopeptide de 2 à 50 acides aminés.

## Claims

1. Anhydrous reagent which is useful especially for cleaving alkoxycarbonyl groups from functional groups which they protect, comprising:
- a diluent A;
- a hydrohalic or pseudohydrohalic acid, the acidity of which is at least equal to that of formic acid,
characterized in that it additionally comprises:
- a phenol B in a ratio by mass with the said diluent of at least 1/200; advantageously of between 1/200th and 1, preferably between 1/20th and 1/2;
and in that the said diluent exhibits the following characteristics:
⇒ capable of dissolving at least 1%, advantageously 2%, of the phenol stricto sensu;
⇒ immiscible in all proportions with water;
⇒ inert.

2. Reagent according to claim 1, characterized in that the said diluent A is such that the water can only dissolve at most 10%, preferably at most 5%, of the said diluent A.

3. Reagent according to claims 1 and 2, characterized in that the said phenol is chosen from the group of compounds corresponding to the following general formula I:
(R₁)ₙ-Ar-O-H (I)
in which:
- Ar represents a heterocyclic or nonheterocyclic, monocyclic or polycyclic aromatic radical;
- the substituents R₁, which are alike or different, represent:
• a halogen, preferably fluorine, chlorine or bromine, unit,
• a group -Z-R₂, where Z can be
- a single bond;
- an oxygen atom;
where R₂ represents a hydrogen atom or an optionally hydroxylated or mono- or polyhalogenated alkyl or aryl radical containing at most 8 carbon atoms,
- n represents the number of substituents and is equal to 0 or to an integer at most equal to the number of positions which can be substituted on the aromatic nuclei; and of their mixtures.

4. Reagent according to one of claims 1 to 3, characterized in that the said phenol B has a number of carbon atoms which is at most 30 carbon atoms and preferably at most 20 carbon atoms.

5. Reagent according to one of claims 1 to 4, characterized in that the vicinal positions of the phenol functional group are unsubstituted or occupied by groups which are not sterically hindering.

6. Reagent according to one of claims 1 to 5, characterized in that the radical -Ar- is a monocyclic aromatic radical having preferably 6 members.

7. Reagent according to claims 1 to 6, characterized in that the said diluent A is chosen from aromatic derivatives, ethers, esters and halogenated solvents.

8. Reagent according to one of claims 1 to 7, characterized in that the hydrohalic acid is chosen from hydrochloric, hydrobromic and hydriodic acids, preferably hydrochloric acid.

9. Process for the treatment of protected molecules, characterized in that the said molecule is protected by an alkoxycarbonyl group and in that the said molecule is subjected to the reagents according to one of claims 1 to 8.

10. Process according to claim 9, characterized in that the said protected molecule is an oligopeptide containing 2 to 50 amino acids.

## Patentansprüche

1. Wasserfreies Reagenz, das insbesondere zum Abspalten von Alkoxycarbonylgruppen von Funktionen, die diese schützen, nützlich ist, umfassend:
- ein Verdünnungsmittel A,
- eine Halogenwasserstoffsäure oder Pseudohalogenwasserstoffsäure, deren Acidität mindestens gleich jener von Ameisensäure ist,
dadurch gekennzeichnet, daß es darüberhinaus umfaßt:
- ein Phenol B in einem Massenverhältnis zu dem Verdünnungsmittel von mindestens 1/200, vorteilhafterweise zwischen 1/200 und 1, vorzugsweise zwischen 1/20 und 1/2,
und dadurch, daß das Verdünnungsmittel die folgenden Eigenschaften aufweist:
- es ist in der Lage, mindestens 1%, vorteilhafterweise 2% des Phenols stricto sensu zu lösen,
- es ist nicht in jedem Verhältnis mit Wasser mischbar,
- es ist inert.

2. Reagenz nach Anspruch 1, dadurch gekenzeichnet, daß das Verdünnungsmittel A dergestalt ist, daß Wasser höchstens 10%, vorzugsweise höchstens 5% des Verdünnungsmittels A lösen kann.

3. Reagenz nach den Ansprüchen 1 und 2, dadurch gekennzeichnet, daß das Phenol ausgewählt wird aus der Gruppe der Verbindungen, die der folgenden allgemeinen Formel I entsprechen:
(R₁)n-Ar-O-H (I),
in der:
- Ar für einen monocyclischen, polycyclischen, heterocyclischen oder nicht heterocyclischen aromatischen Rest steht,
- die Substituenten R₁, die gleich oder verschieden sind, für
- ein Halogenmotiv, vorzugsweise Fluor, Chlor, Brom,
- eine Gruppe -Z-R₂- stehen, wobei Z
- eine Einfachbindung,
- ein Sauerstoffatom sein kann,
wobei R₂ für ein Wasserstoffatom, einen gegebenenfalls hydroxylierten oder einfach oder mehrfach halogenierten Alkyl- oder Arylrest mit höchstens 8 Kohlenstoffatomen steht,
- n für die Anzahl von Substituenten steht und gleich 0 ist oder eine ganze Zahl von höchstens gleich der Anzahl von substituierbaren Positionen an den aromatischen Ringen darstellt, und deren Mischungen.

4. Reagenz nach einem der Ansprüche 1 bis 3, dadurch gekennzeichnet, daß das Phenol B eine Anzahl von Kohlenstoffatomen von höchstens 30 Kohlenstoffatomen, vorzugsweise von höchstens 20 Kohlenstoffatomen hat.

5. Reagenz nach einem der Ansprüche 1 bis 4, dadurch gekennzeichnet, daß die vicinalen Positionen der Phenolfunktion nicht substituiert oder durch nicht sperrige Gruppen besetzt sind.

6. Reagenz nach einem der Ansprüche 1 bis 5, dadurch gekennzeichnet, daß der Rest -Ar- ein monocyclischer aromatischer Ring mit vorzugsweise 6 Ringatomen ist.

7. Reagenz nach den Ansprüchen 1 bis 6, dadurch gekennzeichnet, daß das Verdünnungsmittel A aus Aromaten, Ethern, Estern und halogenierten Lösemitteln ausgewählt wird.

8. Reagenz nach einem der Ansprüche 1 bis 7, dadurch gekennzeichnet, daß die Halogenwasserstoffsäure ausgewählt wird aus Chlorwasserstoffsäure, Bromwasserstoffsäure, Iodwasserstoffsäure, vorzugsweise Chlorwasserstoffsäure.

9. Verfahren zur Behandlung von geschützten Molekülen, dadurch gekennzeichnet, daß das Molekül durch eine Alkoxycarbonylgruppe geschützt ist, und dadurch, daß das Molekül den Reagenzien nach einem der Ansprüche 1 bis 8 unterworfen wird.

10. Verfahren nach Anspruch 9, dadurch gekennzeichnet, daß das geschützte Molekül ein Oligopeptid mit 2 bis 50 Aminosäuren ist.
